# EUROPÄISCHE PATENTANMELDUNG

(11) **EP 1 310 265 A1**
(43) Veröffentlichungstag der Anmeldung: **14.05.2003**
(21) Anmeldenummer: 02025159.1
(22) Anmeldetag: 11.11.2002
(51) Int. Cl.: A61L 9/12, F24F 3/12

(54) **Luftverbessernder Einsatz in Zu- und Abluftanlagen**

(30) Priorität: 12.11.2001 DE 20218329 U
(71) Anmelder: Biothys GmbH, 77964 Kehl (DE)
(72) Erfinder: Wuest, Robert, 67190 Rosheim (FR)
(74) Vertreter: Grussdorf, Jürgen, Dr.

(57) **Zusammenfassung**

Die Erfindung betrifft einen luftverbessernden Einsatz in Zu- und Abluftanlagen in Form einer Hülse (1), die an ihrem zylindrischen Umfang eine Vielzahl von Löchern (2) aufweist, durch welche Luft durchströmen kann, und die mit einem Trägermaterial mit luftverbessernden Essenzen gefüllt ist.

## Beschreibung

Die Erfindung betrifft einen luftverbessernden Einsatz in Zu- und Abluftanlagen, beispielsweise in Klimaanlagen bzw. in Abluft-Kaminen.

Zur Luftverbesserung in Klimaanlagen wird gelegentlich in den Klimaschacht ein luftverbessernder Duftstoff eingesprüht oder auf Sand verteilt eingebracht. Da der Duftstoff von dem Luftstrom mitgerissen wird und somit rasch verdunstet, muss er häufig, meist mehrmals täglich ersetzt werden. Außerdem verbleiben oft Sand-Rückstände im Schacht, so dass dieser häufig gereinigt werden muss. In Kaminen, wo die Abluft geruchsbelästigende Gase und Aerosole enthält, werden diese durch recht aufwendige Absorptions- oder Waschprozesse entfernt.

Der Erfindung lag nun die Aufgabe zugrunde, eine einfache Vorrichtung zur Luftverbesserung in Zu- und Abluftschächten bereitzustellen. Diese Aufgabe wird durch den erfindungsgemäßen Einsatz gelöst. Dieser hat die Form einer Hülse, die an ihrem zylindrischen Umfang eine Vielzahl von Löchern aufweist.

Die Hülse ist vorzugsweise 170 bis 600 mm lang und hat einen Durchmesser von 20 bis 200 mm. Sie weist an ihrem Umfang 100 bis 2000 Löcher mit einem Durchmesser von 2 bis 10 mm auf. Diese Löcher sind vorzugsweise rund; sie können aber auch oval oder vieleckig geformt sein.

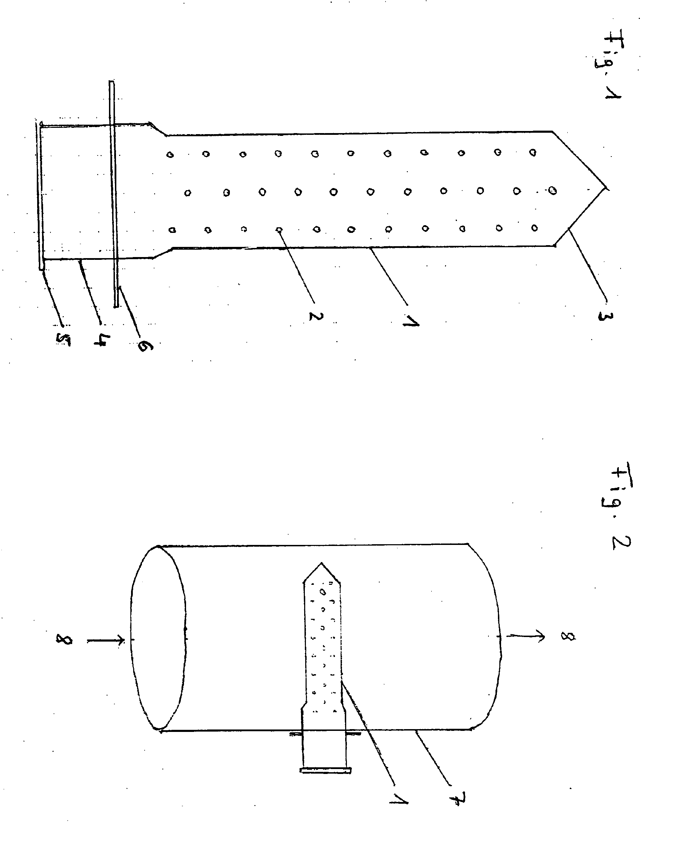

Figur 1 zeigt eine erfindungsgemäße Hülse (1) mit Löchern (2). Das vordere Ende (3) der Hülse ist verschlossen, das hintere Ende (4) ist mit einer Verschlusseinrichtung (5) z. B. einem Deckel, einer Kappe oder einem Stopfen versehen.
Figur 2 zeigt die Hülse im eingebauten Zustand in den Schacht. Sie ist in diesen bis zum Anschlag (6) eingeschoben oder eingeschraubt, der mit einer - nicht eingezeichneten - Dichtungsmasse, beispielsweise aus Silikon, versehen sein kann. Das hintere Ende (4) ragt aus dem Schacht heraus, es weist natürlich keine Löcher auf. Die Hülse ist erfindungsgemäß mit einem Trägermaterial mit den luftverbessernden Essenzen gefüllt, die durch die Verschlusseinrichtung (5) entleert und neu befüllt werden kann. Der Luftstrom (8) durchströmt über die Löcher (2) auch die Hülse und nimmt dabei definierte Mengen der luftverbessernden Essenzen mit. Diese Mengen können durch das Zusammenspiel zwischen der Menge und Geschwindigkeit der durchströmenden Luft, der Anzahl und Größe der Löcher sowie der Art der Essenzen gezielt eingestellt werden. Je nach der notwendigerweise abgegebenen Menge an Essenzen wird der Inhalt der Hülse nach 1 bis 20, vorzugsweise nach 2 bis 10 Wochen ausgewechselt.

Das Trägermaterial besteht vorzugsweise aus einem Vlies, z. B. aus Kunststofffasem oder Naturfasern, man kann aber auch ein textiles Material oder ein saugfähiges Papier zusammengerollt in die Hülse einschieben. Eine weitere Möglichkeit ist die Verwendung einer schwammartigen Polymermatrix, aus der darin verteilten Essenzen heraussublimieren können. 100 Gew. Teile Trägermaterial werden vorzugsweise mit 20 bis 400 Gew. Teilen der Essenzen getränkt, wobei sich dann 20 bis 200, vorzugsweise 30 bis 150 g der wirksamen Essenzen in der Hülse befinden.
In manchen Fällen kann es zweckmäßig sein, zusätzlich ein Fixiermittel für die Essenzen, z. B. auf Basis von Silikaten oder organischen Polymeren, vorzugsweise in Mengen von 2 bis 20 Gew. %, bezogen auf die Essenzen, auf dem Trägermaterial zu verteilen. Auch der Zusatz von Flammschutzmitteln, z. B. Zucker und/oder Bromverbindungen kann zweckmäßig sein.

Die luftverbessernden Essenzen können entweder geruchsneutralisierende Substanzen, z. B. Aldehyde, Alkohole, Ester oder Ketone sein, oder Duftstoffe, wie z. B. Parfüme mit Duft von Fichtennadeln, Blumen, Zitronen oder Leder.

Der erfindungsgemäße Einsatz kann direkt, vorzugsweise horizontal in die Wand eines Zu- bzw. Abluftschachtes eingeschoben sein. Die im Schacht vorbeistreichende Zu- bzw. Abluft bewirkt dann das Verdunsten der luftverbessernden Essenzen. In manchen Fällen, insbesondere wo hohe Schmutz- oder Abriebmengen im Schacht die Einsätze verstopfen könnten, kann es zweckmäßig sein, die Einsätze außerhalb des Schachtes bzw. einer Abluftkammer in einem mit der Kammer verbundenen Ansatz einzubringen. Durch einen Ventilator wird dann in der Kammer ein Unterdruck erzeugt. Dieser bewirkt die Verdunstung der luftverbessernden Essenzen aus dem Einsatz. Diese können sich dann in der Kammer mit der Abluft vermischen und ihre Wirksamkeit entfalten.

Die erfindungsgemäße Einrichtung kann in verschiedenenartigen Anwendungsbereichen eingesetzt werden, z. B. in Küchen, Hotelräumen, Büros, Kaufhäusern, Kinos und Diskotheken, aber auch in Industrieanlagen, wo Abgase anfallen.

## Patentansprüche

1. Luftverbessernder Einsatz in Zu- und Abluftanlagen, **dadurch gekennzeichnet, dass** der Einsatz die Form einer Hülse hat, die an ihrem zylindrischen Umfang eine Vielzahl von Löchern aufweist, durch welche Luft durchströmen kann, und die mit einem Trägermaterial mit luftverbessernden Essenzen gefüllt ist.

2. Einsatz nach Anspruch 1, **dadurch gekennzeichnet, dass** die Hülse eine Länge von 170 bis 600 mm und einen Durchmesser von 20 bis 200 mm aufweist.

3. Einsatz nach Anspruch 1, **dadurch gekennzeichnet, dass** der Einsatz 100 bis 2000 Löcher mit einem Durchmesser von 2 bis 10 mm aufweist.

4. Einsatz nach Anspruch 1, **dadurch gekennzeichnet, dass** die Hülse bevorzugt horizontal in die Wand eines Schachtes eingeschoben ist, wobei ihr hinteres Ende aus diesem herausragt.

5. Einsatz nach Anspruch 1, **dadurch gekennzeichnet, dass** die Hülse an ihrem vorderen Ende verschlossen ist und das hintere Ende mit einer Verschlusseinrichtung zum Befüllen und Entleeren des Trägermaterials versehen ist.

6. Einsatz nach Anspruch 1, **dadurch gekennzeichnet, dass** das Trägermaterial aus einem Faservlies, aus zusammengerolltem textilen Material oder saugfähigem Papier oder aus einer schwammartigen Polymermatrix besteht.

7. Einsatz nach Anspruch 1, **dadurch gekennzeichnet, dass** die luftverbessernden Essenzen geruchsneutralisierende Substanzen oder Duftstoffe sind.
